# EUROPEAN PATENT APPLICATION

(11) **EP 0 845 217 A1**
(43) Date of publication of application: **03.06.1998**
(21) Application number: 97309034.3
(22) Date of filing: 11.11.1997
(51) Int. Cl.: A23L 1/211, A61K 31/66, A61K 31/28, A23L 1/03

(54) **Inhibiting undesirable taste in oral compositions**

(30) Priority: 20.11.1996 US 752088
(71) Applicant: THE PROCTER & GAMBLE COMPANY, Cincinnati, Ohio 45202 (US)
(72) Inventor: Nelson, Sandra Lynn, Loveland, Ohio 45140 (US); Walden, Gary Lyle, West Chester, Ohio 45069 (US); Hayes, Jeffrey Charles, West Chester, Ohio 45069 (US)
(74) Representative: Woof, Victoria

(57) **Abstract**

The present invention relates to a method for inhibiting an undesirable taste in oral compositions such as foods, beverages, dental compositions, and pharmaceuticals. The present invention also relates to oral and pharmaceutical compositions comprising undesirable tasting compounds wherein undesirable tastes are inhibited by the addition of an inhibitor of intracellular phosphatase enzymes of taste cells to said oral and pharmaceutical compositions. Said inhibitor is selected from phosphates, thiophosphates, phosphonates, vanadates, bisphosphates, bisphosphonates, phosphate-phosphonates, thiophosphate-phosphates, thiophosphate-phosphonates, their physiologically-relevant salts and esters, and mixtures thereof.

## Description

### TECHNICAL FIELD

The present invention relates to a method for inhibiting an undesirable taste in oral compositions such as foods, beverages, and pharmaceuticals. The method involves the addition of an inhibitor of intracellular phosphatase enzymes of taste cells to said compositions.

### BACKGROUND OF THE INVENTION

Consumers do not care for the taste modality of bitterness in the broadest sense. Therefore, the desire for improved palatability of oral compositions having a bitter taste has prompted the development of numerous formulations and methods of bitterness reduction/inhibition.

In most cases, the consumer or producer have attempted to mask the bitter attributes of various oral compositions by using compounds or mixtures of compounds, such as flavors and sugars or other sweetening ingredients. Gelatin (Japanese Patent Application No. 04-346,937); gelatinized starch (Japanese Patent Application No. 04-235,136); acidic amino acids (U.S. Patent No. 4,517,379 and Japanese Patent Application Nos. 05-015,389 and JP 05-004,921); chitosan (Japanese Patent Application No. 04-009,335); cyclodextrins (U.S. Patent No. 5,024,997); liposomes (U.S. Patent No. 5,009,819); lecithin or lecithin like substances (Japanese Patent Application No. 62-265,234); surfactants (U.S. Patent No. 5,439,671); salts (U.S. Patent No. 5,262,179); and the like have also been used to mask unpleasant tastes in oral compositions. Likewise, coating or microencapsulation (European Patent Application No. 551,820); functional group alteration (U.S. Patent No. 5,350,839); and structural matrix forms of taste masking have been used. Oral compositions employing such technology have incorporated agents such as silicate clays (U.S. Patent Nos. 3,140,978 and 4,581,232); acrylic acid copolymers (U.S. Patent No. 5,286,489); gums (U.S. Patent No. 5,288,500); cellulose (U.S. Patent No. 5,192,563); and waxes in an effort to further provide improved tasting compositions. Unfortunately, masking bitter taste with such compounds, alters the true overall flavor of the oral compositions and not just the bitter taste modality.

Other methods of inhibiting/reducing bitter taste have also been disclosed. For example, Kurtz and Fuller have disclosed (U.S. Patent No. 5,232,735 and PCT Application No. WO 93/10677) modifying certain compounds, such that they become tasteless, which when added to a composition comprising an undesirable taste, the undesirable taste is blocked. Roy, et al., have disclosed N-(sulfomethyl)-N'-arylureas as sweetness and bitterness inhibitors (U.S. Patent Nos. 4,994,490 and 5,266,717). Guadagni, et al. have found that the addition of the flavone, neodiosmin, results in reduced bitterness and off-after-taste (U.S. Patent No. 4,154,862), while Riemer has discovered that certain cinnamic acid derivatives have the ability to inhibit the taste of bitter compounds and the after-taste of artificial sweeteners (U.S. Patent No. 5,336,513). Magnolato has claimed a process for removing bitter tasting compounds from fruit and vegetable extracts by selective absorption using an inexpensive natural ligneous material (U.S. Patent No. 4,282,264) and Miller has used steam to remove the bitter tasting compounds from soybeans (U.S. Patent No. 4,248,141).

An ideal solution to bitterness reduction would be the development of a universal bitterness inhibitor that does not affect the overall flavor of the oral compositions. Katsuragi and Kurihara have reported in *Nature,* vol. 365, pp. 213-214, 1993, a bitterness inhibitor made of phosphatidic acid and β-lactoglobulin which suppresses taste responses and sensations to bitter substances without affecting the responses to other taste stimuli. Unfortunately, such a compound has only shown limited success in inhibiting the bitter taste in many different bitter tasting compounds.

The main process of taste perception involves a recognition of a sapid substance on the surface of the tongue and conversion of the recognition event into a nerve impulse in the appropriate gustatory axon(s). The recognition step often involves the binding of a ligand (i.e. taste molecule) to a receptor on the surface of the taste cell. The taste cells themselves are polarized neuroepithelial cells which are clustered into groups of about fifty cells. These clusters are embedded in the surrounding keratinized squamous epithelium of the taste buds located mainly in the epithelium of the tongue. The apical surface of a taste cell is exposed to the surface of the tongue through a small opening in the taste bud, known as the taste pore, through which taste molecules pass.

It is thought that there are different biochemical pathways which take place within taste cells that are specific to the perception of different taste modalities. For bitter taste, it is thought that one way the biochemical cascade is triggered is when a receptor, which is believed to be G-protein-linked, binds its specific taste ligand. Taste-receptor binding then activates the enzyme phospholipase C (PLC) which acts upon phosphatidylinositol-4,5-bisphosphate (PIP₂) to produce 1D-*myo*-inositol 1,4,5-triphosphate (IP₃). The rise in IP₃ concentration causes an increase in the intracellular Ca²⁺ level via release of Ca²⁺ stores. The Ca²⁺ increase causes a release of neurotransmitter from the taste cell onto a gustatory afferent neuron connected to the lower brain stem. The neural pattern which is generated from the summated response of numerous taste cell firings ultimately results in the perception of bitter taste.

Phosphorylation and dephosphorylation reactions are important biochemical conversions which are used in the regulation of taste transduction processes. The state of phosphorylation of ion channels, key intermediary enzymes, as well as the G-protein-coupled receptors, may contribute in modulating the taste response. For the G-protein-coupled receptors, receptive (sensitized) and nonreceptive (desensitized) states have been found to depend respectively on whether the protein is phosphorylated or not.

The present inventors have surprisingly discovered that certain phosphatase enzyme inhibitors inhibit the phosphatase activity intracellularly, and that the addition of these inhibitors of intracellular phosphatase enzymes to oral compositions having an undesirable taste, such as bitter and/or metallic taste, inhibits said taste without substantially affecting the overall desirable flavor of the compositions.

It is therefore an object of the present invention to provide a method for inhibiting the undesirable taste of oral compositions, such as foods, drinks, over-the-counter and prescription pharmaceuticals, and toiletries, by the addition of an inhibitor of intracellular phosphatase enzymes of taste cells to said compositions.

This and other objects of the invention will become readily apparent from the detailed description which follows.

### SUMMARY OF THE INVENTION

The present invention relates to a method for inhibiting undesirable taste, such as bitter and/or metallic taste, comprising the addition of a safe and effective amount of an inhibitor of intracellular phosphatase enzymes of taste cells to oral compositions such as foods, drinks, pharmaceuticals, and toiletries.

All percentages and ratios used herein are by weight of the total composition, and all measurements made at 25°C, unless otherwise specified.

### DETAILED DESCRIPTION OF THE INVENTION

The present invention comprises a method for inhibiting undesirable taste by adding a safe and effective amount of an inhibitor of intracellular phosphatase enzymes of taste cells to oral compositions such as foods, drinks, pharmaceuticals, and toiletries.

The phrase "oral compositions", as used herein, is defined as any product which in the ordinary course of usage is intentionally swallowed, such as foods, drinks, over-the-counter and/or prescription pharmaceuticals, and the like. However, the phrase "oral compositions", as used herein, is also intended to include the phrase "dental compositions", which as used herein, means any composition used in the oral cavity and/or to clean the teeth, and which is intentionally not swallowed but rather retained in the oral cavity for a time and then substantially expectorated, such as gums, toothpastes, dentifrices, mouthwashes, and the like.

The phrase "undesirable taste", as used herein, is not limited by the basic tastes of sweet, sour, bitter, umami, and salty; but is defined as any taste, including sweet, bitter, sour, alkaline, astringent, tangy, dry, sharp, cool, hot, burning, acidic, spicy, pungent, woody, smoky, umami, metallic, and/or any aftertaste, if such taste is unwanted in a composition.

The term "inhibit", as used herein, is defined as the slowing or interference of the taste transduction mechanism such that, while an undesirable tasting compound remains unchanged within a composition, the perception of the undesirable taste of the compound is decreased in the person or animal consuming said composition.

The term "mask", as used herein, is defined as covering, disguising, and/or obscuring an undesirable taste by the addition of compounds such as sweeteners, flavors, and the like, to compositions having an undesirable tasting compound, wherein the undesirable tasting compound remains unchanged, but its taste is overwhelmed by the other tastes present in the composition such that the undesirable taste is not perceived by the person or animal consuming said composition.

The phrase "chemical alteration", as used herein, is defined as the structural modification of a chemical compound having an undesirable taste such that the compound no longer has as undesirable taste.

The phrase "safe and effective amount", as used herein, is defined as an amount of a substance sufficient to provide the desired benefit without undue adverse side effects, such as toxicity, irritation, or allergic response, commensurate with a reasonable benefit/risk ratio when used in the manner of this invention.

The term "compatible", as used herein, is defined as that the components of the compositions are capable of being co-mingled with one another without substantially reducing the efficacy of the components or the composition under ordinary use conditions.

The term "dentifrice", as used herein, is defined as any composition, including toothpowders, toothpastes, toothgels, or liquids, which are generally used when brushing the teeth.

The phrase "pharmaceutically-acceptable carrier materials" , as used herein, means one or more compatible solid or liquid filler diluents or encapsulating substances which are suitable for oral and/or nasal administration to a human or lower animal. Pharmaceutically-acceptable carrier materials must, of course, be of sufficiently high purity and sufficiently low toxicity to render them suitable for administration to the human being treated.

The phrase "orally-acceptable carrier materials", as used herein, means any material safe and effective for use in the dental compositions of the present invention. Such materials include but are not limited to, thickening materials, humectants, water, buffering agents, abrasive polishing materials, sodium bicarbonate, titanium dioxide, surfactants, flavors, sweeteners, coloring agents, coolants, and mixtures thereof.

### Method Of Inhibiting Undesirable Taste

Within the scope of the present invention the inhibitor of intracellular phosphatase enzymes of taste cells operate by inhibiting the perception of bitter and/or metallic taste. Thus, when the inhibitor of intracellular phosphatase enzymes of taste cells is added to oral compositions containing compounds which cause bitter and/or metallic taste, the oral compositions taste less bitter/metallic than similar oral compositions without the inhibitor of intracellular phosphatase enzymes of taste cells.

Intracellular phosphatase inhibitors which are useful in the present invention include compounds which contain one or more functional groups attached covalently or ionically to at least one other atom. The functional groups have the following chemical structure: 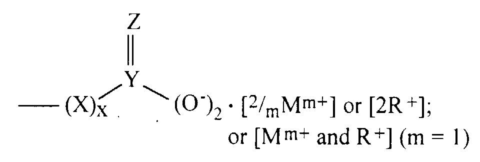 where:
X=O or S; x=0 or 1
Y = P or V
Z = O or S
M = Li, Na, K, NR₄ (m = 1); Mg, Ca, Sn, Cu, Fe, Zn (m = 2)
R = H, alkyl, alkenyl, aryl (including heteroatom-containing aromatics such as pyridyl, etc.), or substituted alkyl, alkenyl, and aryl.

The following compounds are illustrative of the compounds which conform to the above chemical structure and may be useful in the present invention:

Phosphates, including their physiologically-relevant salts and ester derivatives. Examples include vanillylmonophosphate (VMP), eugenylmonophosphate (EMP), menthylmonophosphate (MMP), thymylmonophosphate (ThMP), phosphotyrosine, β-napthyl phosphate, α-napthyl phosphate, phenyl phosphate, 4-nitrophenylphosphate, 3-nitrophenylphosphate, 2-chlorophenyl phosphate, D-inositol-1-phosphate, D-3,5,6-trideoxy-inositolphosphate disodium, D-6-deoxy-inositolphosphate disodium, α-napthylyl acid phosphate monosodium, vanillyl monophosphate monosodium, ethyl vanillyl monophosphate monosodium, and mixtures thereof.

Thiophosphates, including their physiologically-relevant salts and ester derivatives. Examples include thio-MMP, *rac*-inositol 1-phosphorothioate, thiophosphoryltyrosyl-containing peptides, and mixtures thereof.

Phosphonates, including their physiologically-relevant salts and ester derivatives. Examples include phosphonoacetic acid, 2-amino-4-phosphonobutyric acid, benzylphosphonic acid, phosphonomethylphenylalanine, *rac*-inositol 1-phosphonate, D-inositol 1-methylphosphonate, 3,4-methylenedioxybenzylphosphonic acid monosodium, bis(acetoxymethyl)benzylphosphonate, and mixtures thereof.

Vanadates, including their physiologically-relevant salts and ester derivatives. Examples include trisodium orthovanadate.

Bisphosphates, including their physiologically-relevant salts and ester derivatives. Examples include *rac*-Inositol 4,5-bisphosphate, *rac*-Inositol 3,4-bisphosphate, and mixtures thereof.

Bisphosphonates, including their physiologically-relevant salts and ester derivatives. Examples include pamidronic acid, hydroxymethylenebisphosphonic acid, methylene bisphosphonic acid, alendronic acid, azacycloheptane-2,2-diphosphonic acid, *p*-hydroxyphenyl-1-[1,1-ethylenebisphosphonate] ether trisodium, and mixtures thereof.

Mixed classes such as phosphate-phosphonates, thiophosphate-phosphates, and thiophosphate-phosphonates, including their physiologically-relevant salts and ester derivatives. Examples include D-inositol 1,4-bisphosphate-5-methylenephosphonate, *rac*-1,4,5S-IP₃, and mixtures thereof.

Other useful intracellular phosphatase inhibitors which do not conform to this functional group description are: canthardic acid, endothall, and dephostatin or 2-(N-methyl-N-nitroso)hydroquinone.

While these compounds are useful inhibitors of intracellular phosphatase enzymes of taste cells, other compounds which act as inhibitors of phosphatase enzymes known to one skilled in the art, may also be useful in inhibiting undesirable taste.

The amount of the inhibitor of intracellular phosphatase enzymes of taste cells to be added to the oral compositions depends on various factors such as the nature of the composition, the concentration therein of the compound or compounds responsible for the undesirable taste, and the degree of inhibition desired. However, it has been found that useful results are typically obtained by adding from about 0.001% to about 10% by weight, preferably from about 0.01% to about 8% by weight, more preferably from about 0.1% to about 5% by weight, and most preferably from about 0.5% to about 2% by weight, of the inhibitor of intracellular phosphatase enzymes of taste cells to the oral compositions having an undesirable taste.

The oral compositions having an undesirable taste for which inhibition is desired are prepared simply by incorporating the inhibitor of intracellular phosphatase enzymes of taste cells therewith. The methods for inhibiting undesirable taste by the addition of the inhibitor of intracellular phosphatase enzymes of taste cells of the present invention are further described below with reference to foods, drinks, and pharmaceuticals, respectively.

Examples of foods having an undesirable or bitter taste include, but are not limited to, citrus fruits such as grapefruit, orange, and lemon; vegetables such as tomato, pimento, celery, melon, carrot, potato, and asparagus; seasoning or flavoring materials such as flavor, sauces, soy sauce, and red pepper; foods originating from soybean; emulsion foods such as cream, dressing, mayonnaise, and margarine; processed marine products such as fish meat, ground fish meat, and fish eggs; nuts such as peanuts; fermented foods such as fermented soybean; meats and processed meats; pickles; noodles; soups including powdery soups; dairy products such as cheese; breads and cakes; confectioneries such as candies, chewing gum, and chocolate; and specifically prepared foods for health.

Examples of the drinks having an undesirable or bitter taste include juices of citrus fruits and vegetables, soybean milk, coffee, cocoa, black tea, green tea, fermented tea, semi-fermented tea, refreshing drinks, beverages, and milk.

Examples of pharmaceutical compositions having an undesirable or bitter/metallic taste include, but are not limited to, cough/cold oral compositions, dental compositions and compositions for treating the oral cavity, and compositions for treating gastrointestinal distress. There are no specific limitations with respect to the orally administrable pharmaceutical actives whose undesirable tastes can be suppressed by addition of the inhibitor of intracellular phosphatase enzymes of taste cells of the invention. However, these pharmaceutically acceptable actives should be compatible with the inhibitor of intracellular phosphatase enzymes of taste cells of the present invention, as well as other essential ingredients in the oral compositions. The inhibitor of intracellular phosphatase enzymes of taste cells of the present invention may also be used with mixtures of various pharmaceutical actives. Typical examples of the pharmaceutically acceptable actives which have an undesirable taste include actives selected from among the various groups of chemical compounds or materials suitable for oral administration and having a pharmacological action.

Pharmaceutically acceptable active materials or compounds useful for treating cough, cold, cold-like, allergy and/or flu symptoms and which have an undesirable taste may include, but are not limited to, an active having analgesic, anti-inflammatory, antipyretic, anesthetic, antihistamine, bronchodilators, decongestant, cough suppressant, demulcents, antitussive, and/or expectorant properties. These actives, as well as their acceptable dosage ranges are described in U.S. Patent 4,783,465 to Sunshine et al., issued November 8, 1988; U.S. Patent 4,619,934 to Sunshine et al., issued October 28, 1986; and Remington's Pharmaceutical Sciences, pp. 734-789, 791-799, 861-868, 907-945, 875-888, 1002-1034, 1098-1121, 1124-1131, 1173-1224, 1232-1241 (Alfonso R. Gennaro, editor) (18th ed. 1990), all of which are incorporated by reference herein.

Examples of said decongestants having an undesirable taste include pseudoephedrine, phenylpropanolamine, phenylephrine and ephedrine, their pharmaceutically acceptable salts, and mixtures thereof.

Examples of said antitussives having an undesirable taste include dextromethorphan, chlopedianol, carbetapentane, caramiphen, noscapine, diphenhydramine, codeine, hydrocodone, hydromorphone, their pharmaceutically acceptable salts, and mixtures thereof.

Examples of said expectorants (also known as mucolytic agents) having an undesirable taste include guaifenesin, terpin hydrate, ammonium chloride, N-acetylcysteine, and ambroxol, their pharmaceutically acceptable salts, and mixtures thereof.

Examples of said analgesics having an undesirable taste include morphine, codeine, meperidine, pentazocine, propoxyphene, acetaminophen, allopurinol, acetylsalicylic acid, choline salicylate, ketoprofen, magnesium silicate, fenoprofen, ibuprofen, flurbiprofen, indomethacin, naproxen, and many others, their pharmaceutically acceptable salts, and mixtures thereof.

Examples of said antihistamines having an undesirable taste include brompheniramine, chlorpheniramine, clemastine, dexchlorpheniramine, diphenhydramine, doxylamine, promethazine, terfenadine, triprolidine, and many others, their pharmaceutically acceptable salts, and mixtures thereof.

Dental actives having an undesirable taste typically comprise anticalculus agents, tartar control agents, anticaries agents, antimicrobial/antiplaque agents, antibacterial agents, anti-inflammatory agents, surfactants, nutrients, and the like. Examples of said anticalculus agents include, but are not limited to, those disclosed in U.S. Patent No. 4,590,066 issued to Parran & Sakkab on May 20, 1986; U.S. Patent No. 3,429,963 issued to Shedlovsky on February 25, 1969, and U.S. Patent No. 4,304,766 issued to Chang on December 8, 1981, and U.S. Patent No. 4,661,341 issued to Benedict & Sunberg on April 28, 1987; U.S. Patent No. 4,846,650 issued to Benedict, Bush & Sunberg on July 11, 1989; British Patent No. 490,384 dated February 15, 1937; U.S. Patent No. 3,678,154 issued to Widder & Briner on July 18, 1972; U.S. Patent No. 3,737,533 issued to Francis on June 5, 1973, U.S. Patent No. 3,988,443 issued to Ploger, Schmidt, Dunker & Gloxhuber on October 26, 1976, and U.S. Patent No. 4,877,603 issued to Degenhardt & Kozikowski on October 31, 1989; all of these patents are incorporated herein by reference.

Examples of said anticaries agents having an undesirable taste include, but are not limited to, water-soluble fluoride ion sources. The number of such fluoride ion sources is great and includes sodium fluoride, potassium fluoride, indium fluoride, and sodium monofluorophosphate and mixtures thereof, as well as those disclosed in U.S. Patent No. 2,946,735 issued July 26, 1960 to Norris, et al., U.S. Patent No. 3,535,421 issued October 20, 1970 to Briner & Widder, and U.S. Patent No. 3,678,154 issued July 18, 1972 to Widder, et al., all of which are incorporated herein by reference.

Examples of said antimicrobial/antiplaque agents having an undesirable taste include, but are not limited to, noncationic water insoluble antimicrobial agents such as halogenated diphenyl ethers, phenolic compounds including phenol and its homologs, mono and poly-alkyl and aromatic halophenols, resorcinol and its derivatives, bisphenolic compounds and halogenated salicylanilides, benzoic esters, and halogenated carbanilides, as well as those described in The Merck Index, 10th ed. (1976), U.S. Patent No. 3,506,720, and European Patent Application No. 0,251,591 of Beecham Group, PLC, published January 7, 1988, U.S. Patent No. 4,670,252, U.S. Patent No. 5,338,537, issued August 8. 1994, to White, et al., and U.S. Patent No. 5,389,360, issued February 14, 1995, to Mobley, et al., all of which are incorporated herein by reference in their entirety.

Examples of antibacterial agents having an undesirable taste include, but are not limited to, noncationic and substantially water insoluble antibacterial agents.

Examples of said anti-inflammatory agents having an undesirable taste include, but are not limited to, aspirin, ibuprofen, naproxen, indomethacin, piroxicam, flurbiprofen, meclofenamate sodium, ketoprofen, tenidap, tebufelone, ketorolac, and the like.

Examples of said nutrients having an undesirable taste include, but are not limited to, folate, retinoids (Vitamin A), Vitamin C, Vitamin E and zinc.

Pharmaceutical actives, having an undesirable taste, useful for treating upper gastrointestinal tract distress include, but are not limited to, antacid agents and acid secretion prevention agents (e.g., H2 receptor-blocking anti-secretory agents) such as aluminum carbonate, aluminum hydroxide, aluminum phosphate, aluminum hydroxy-carbonate, dihydroxy aluminum sodium carbonate, aluminum magnesium glycinate, dihydroxy aluminum amino acetate, dihydroxy aluminum aminoacetic acid, calcium carbonate, calcium phosphate, aluminum magnesium hydrated sulfates, magnesium aluminate, magnesium alumino silicates, magnesium carbonate, magnesium glycinate, magnesium hydroxide, magnesium oxide, magnesium trisilicate, sucralfate, cimetidine, ranitidine, famotidine, omeprazole, bismuth-containing agents such as bismuth subsalicylate, bismuth aluminate, bismuth citrate, bismuth subcitrate, bismuth nitrate, bismuth subcarbonate, bismuth subgalate, and mixtures thereof. Anticholinergics such as atropine, clidinium and dicyclomine, laxatives such as phenolphthalein and casanthrol, and antidiarrheals such as diphenoxylate and loperamide may also be included.

The undesirable taste of other basic pharmaceutically active acid addition salts such as strychnine, quinine, papaverine, berberine, promethazine, brucine, propranolol, and chlorpromazine may also be suppressed by addition of the inhibitor of intracellular phosphatase enzymes of taste cells of the invention. Inorganic acid salts and organic acid salts such as hydrochloride, nitrate, sulfate, acetate, citrate, and carbonate of basic pharmaceutically active components may also be included. The pharmaceuticals can be in any preparation forms such as solid preparations (e.g., capsule, granules, medicinal pill, powder, pellet, troche and dry syrup); and liquid preparations (e.g., liquids, extracts, elixirs, spirits, syrups, aromatic water, lemonades, and fluid-extracts). The inhibitor of intracellular phosphatase enzymes of taste cells can be incorporated into the pharmaceutical preparation in any conventional manner. For instance, the inhibitor of intracellular phosphatase enzymes of taste cells can be incorporated into the pharmaceutical preparation singly or in combination with one or more of known additives. Examples of such known additives include, but are not limited to, diluent, filler, excipient, vehicle, binder, disintegrator, lubricant, fluidity-improving agent, coating agent, flavor, masking agent, perfume, and anti-oxidation agent. The pharmaceutical preparation can be produced using any conventional means. Typically, inhibition of undesirable taste is achieved by the addition of the inhibitor of intracellular phosphatase enzymes of taste cells comprises from about 0.001% to about 10% by weight, preferably from about 0.01% to about 8% by weight, more preferably from about 0.1% to about 5% by weight, and most preferably from about 0.5% to about 2% by weight, of pharmaceutical compositions having an undesirable taste.

The inhibitor of intracellular phosphatase enzymes of taste cells can be coated on a composition having a bitter taste. For instance, foods in the form of a solid, such as candy, other confectioneries, processed fish/meats, vegetables, fruits, processed vegetables, processed fruits, dried vegetable juices, and dried fruits juices and pharmaceutical preparations in the form of powder, granules, pellets, tablets, soft and hard capsules, and pills. The coating layer can comprise the inhibitor of intracellular phosphatase enzymes of taste cells and hydrophilic polymers such as cellulose derivatives, gelatin, and polyvinyl alcohol. Other additives such as sweeteners and flavors can be incorporated into the coating layer. There is no need of coating the whole surface of the material. Partial coating may also be employed. The coating comprising the inhibitor of intracellular phosphatase enzymes of taste cells can be made on the composition which already contains the inhibitor of intracellular phosphatase enzymes of taste cells. For the coating, any known coating methods and coating apparatuses can be used.

### EXAMPLES

The following examples further describe and demonstrate the inhibition of intracellular phosphatase enzymes of taste cells. These examples are given solely for the purpose of illustration and are not to be construed as a limitation of the present invention as many variations thereof are possible without departing from the spirit and scope of the present invention.

### Example 1

This example demonstrates phosphatase enzyme inhibitors.

The enzyme activity of alkaline phosphatase (a serine-threonine phosphatase), acid phosphatase (a serine-threonine phosphatase) and protein tyrosine phosphatase is inhibited by at least one flavor phosphate, as well as other inhibitors that are known in the scientific literature. Alkaline phosphatase is competitively inhibited by VMP, EMP, MMP, ThMP, and phosphoserine. This enzyme exhibits classic Michaelis-Menten kinetics. Enzyme kinetics experiments are done using purified alkaline phosphatase from bovine intestine and the substrate *para*-nitrophenyl phosphate (PNPP). The formation of colored para-nitrophenol (PNP) from colorless PNPP is followed by measuring the color formation in the presence of inhibitor at several concentrations.

Specifically, enzyme reactions are done in a volume of 200 ul containing 12.5 mU/ml alkaline phosphatase, PNPP at concentrations between 0.5-200 mM, and an inhibitor at concentrations between 0-100 mM. The inhibitors are: VMP, EMP, MMP, ThMP, and phosphoserine. Phosphoserine is the natural substrate for this enzyme. The final buffer solution is 0.9 M diethanolamine and 0.5 mM HgCl₂, pH 9.8, mixed 3:1 (v:v) with water. Reactions are done in a 96-well microtiter plate. The enzyme reaction is initiated by the addition of the enzyme. Each reaction mixture is done in triplicate. The enzyme reaction is monitored by measuring the optical density (OD) at 405 nm, with a reference OD of 490 nm. The OD's are read every 30 seconds over a 15 minute period at room temperature. PNP concentration is determined based on a standard curve correlating OD_{405/490} vs. PNP concentration. Linear enzyme reaction rates are observed in the concentration ranges tested. For each inhibitor, plots of ¹/_{S} vs. ¹/_{V} demonstrates competitive inhibition, with no change in V_{max.} but a change in the apparent Michaelis-Menten constant, K_{mapp.} The data is then transformed and plotted as the Kₘₐₚₚ / V_{max.} vs. inhibitor concentration. From these plots the dissociation constant of the enzyme-inhibitor complex, Kᵢ, is determined. The x-intercept on this plot equals (-Kᵢ). The Kᵢ values determined in this way are shown in Table 1 below. The data shows that EMP is the best inhibitor of the phosphatase, while MMP and VMP are equivalent to the natural substrate, phosphoserine. ThMP however, is a less effective competitive inhibitor of the phosphatase than the other compounds tested.

**TABLE 1**

| Kᵢ Values of Inhibitors with Alkaline Phosphatase and PNPP Substrate | |
|---|---|
| **Inhibitor** | **K**_{**i**} |
| EMP | 0.877 mM |
| Phosphoserine | 3.75 mM |
| MMP | 3.91 mM |
| VMP | 3.99 mM |
| ThMP | 5.19 mM |

Acid phosphatase, another serine-threonine phosphatase, is shown to be competitively inhibited by VMP. Enzyme kinetics are done using purified acid phosphatase (EC 3.1.3.2) type V from bovine milk with PNPP as the substrate. The enzyme kinetics experiments are done similarly to the kinetics experiments for alkaline phosphatase, described above.

Specifically, each enzyme reaction is done in a 200 ul volume containing 500 ug/ml (500 mU/ml) acid phosphatase, PNPP at concentrations between 1.25-50 mM and VMP at concentrations between 0-80 mM. VMP is the only inhibitor tested with acid phosphatase. The buffer is 0.2 M sodium acetate buffer, pH 5.6. Reactions are done in a 96-well microtiter plate. The enzyme reaction is initiated by the addition of enzyme. The enzyme reaction is stopped by the addition of 50 ul of 3 N NaOH to each well. Reaction times are from 0-15 minutes, at 30 second intervals. As described previously, the reaction rate is determined by measurement of the formation of colored product, PNP, which correlates with OD_{405/490}. Acid phosphatase has a linear enzyme reaction rate for 15 minutes with the concentration ranges tested. Calculation of the Kᵢ is done as described above. The Kᵢ for VMP with acid phosphatase and PNPP substrate at room temperature is 9.16 mM. The data demonstrates that VMP is a competitive inhibitor of PNPP.

Protein tyrosine phosphatase, a tyrosine phosphatase, is shown to be inhibited by VMP, EMP, and ThMP. Enzyme kinetics are done using purified protein tyrosine phosphatase from the bacteria, Yersinia enterocolitica, recombinant form, with PNPP as the substrate. The enzyme kinetics experiments are done similarly to the kinetics experiments for alkaline phosphatase and acid phosphatase described above.

Specifically, the enzyme reaction is done in a volume of 200 ul containing 120 ng of tyrosine phosphatase, 0.2-1.0 mM PNPP, and 0-0.5 mM VMP, EMP or ThMP. Each final reaction mixture contains 20 mM Tris, 0.1 % beta-mercaptoethanol, and 0.1% bovine serum albumin at pH 7.5. The ionic strength of each reaction mixture is the same and is balanced by the addition of the appropriate amount of NaCl. Activity of this enzyme is extremely sensitive to ionic strength. A 96-well microtiter plate is used for the enzyme reactions and the reactions are done at the temperatures indicated in Table 2. Each reaction is begun with the addition of tyrosine phosphatase. The formation of PNP is monitored by measuring the OD_{405/490} at 30 second intervals over a 30 minute period. The enzyme velocity is linear over this time period under these conditions. The Kᵢ values are calculated as described above for alkaline phosphatase (Table 2).

**TABLE 2**

| Kᵢ Values of Inhibitors with Protein Tyrosine Phosphatase and PNPP Substrate | | |
|---|---|---|
| **Inhibitor** | **K**_{**i**} | **Temperature** |
| VMP | 1.04 mM | 37°C |
| EMP | 3.3 mM | 37°C |
| EMP | 3.7 mM | 22°C |
| ThMP | 3.6 mM | 22°C |

In other experiments (not described herein), phosphotyrosine is shown to inhibit the dephosphorylation of phosphotyrosine-containing peptide substrates by purified protein tyrosine phosphatase. As controls in these experiments, sodium orthovanadate, an inhibitor of protein tyrosine phosphatase, inhibits the tyrosine phosphatase activity. Calcium fluoride, an inhibitor of serine-threonine phosphatase enzymes, does not affect tyrosine phosphatase activity. VMP, EMP and phosphotyrosine also inhibits the activity of purified tyrosine phosphatase against the peptide substrates.

### Example 2

This example demonstrates the presence of phosphatase activity in taste tissue.

Serine-threonine type phosphatase activity is present in all cells containing G-protein linked receptor signal transduction mechanisms (C.J. Pallen et al, *Current Opinion in Cell Biology* 4: 1000-1007, 1992). More specifically, serine-threonine phosphatase enzymes play a role in the G-protein linked signal transduction mechanisms of olfactory cells (H. Breer, *The Molecular Basis of Smell and Taste Transduction,* (Ciba Foundation Symposium 179) pp. 97-114, eds. D. Chadwick et al., Wiley, 1993). Olfactory cells are also chemoreceptor cells and are similar to taste cells. Therefore, precedence of serine-threonine phosphatases in taste cells is well established in the scientific literature.

Protein tyrosine phosphatase in taste cells is demonstrated in the following experiments. Taste tissue (circumvallate papillae) is dissected from fresh pig tongue and homogenized in buffer. Protein tyrosine phosphatase activity is measured using peptides containing phosphotyrosine as the substrate. Two substrates are used. Each contain one phosphotyrosine residue; peptide substrate 1 has 13 amino acids and peptide substrate 2 has 17 amino acids. The removal of phosphate from the phosphotyrosines is determined by measurement of phosphotyrosine concentration, using an ELISA, after reaction of the peptide substrate with pig taste tissue. The activity of purified tyrosine phosphatase is determined in each experiment as a control.

In this experiment, pig taste tissue and peptide substrate are combined in a buffer of 20 mM Tris, 0.1% beta-mercaptoethanol, and 1 mg/ml BSA, pH 7.0. A total volume of 100 ul in each well of a 96-well microtiter plate is used. Sodium orthovanadate, calcium fluoride and VMP are added to some reaction mixtures to determine their ability to inhibit the enzyme reaction. The enzyme reaction is done at 10°C for 20 min. The reaction is stopped by the addition of 25 ul 400 uM sodium orthovanadate. A dose-response of tyrosine phosphatase activity in pig taste tissue is observed (Table 3).

**TABLE 3**

| Tyrosine Phosphatase Activity in Pig Taste Tissue | |
|---|---|
| **Pig Taste Tissue (µg/ml protein)** | **Substrate Remaining (pmoles phosphotyrosine/ml)** |
| 0.0 | 1000 |
| 38.4 | 52 |
| 77.9 | 36 |
| 154.0 | 22 |
| 307.5 | 15 |
| 615.0 | 1 |

In this experiment (Table 4), enzyme activity of pig taste tissue is inhibited by sodium orthovanadate, an inhibitor of protein tyrosine phosphatase but is not inhibited by fluoride, an inhibitor of serine-threonine phosphatases. Pig taste tissue tyrosine phosphatase dephosphorylated peptide substrate 1 faster than peptide substrate 2.

**TABLE 4**

| Protein Tyrosine Phosphatase Activity of Pig Taste Tissue (615 ug/ml protein) | | |
|---|---|---|
| | **% Total Phosphatase Activity** | |
| **Inhibitor** | **Peptide Substrate 1** | **Peptide Substrate 2** |
| none | 100 | 100 |
| sodium orthovanadate | 32 | 46 |
| calcium fluoride | 109 | 96 |

In another experiment using the same conditions as described above, the tyrosine dephosphorylation of peptide substrate 1 is measured after 1, 2, 3, 4, 5, 6, 7 and 8 minute reactions at room temperature with pig taste tissue (41 ug/ml protein) as the enzyme source. All phosphates are removed from the substrate after 2 minutes by pig taste tissue tyrosine phosphatase. Addition of calcium fluoride (a serine-threonine phosphatase inhibitor) does not change the dephosphorylation rate; all phosphates are removed after 2 minutes Addition of sodium orthovanadate to a final concentration of 100 nmoles/ml or phosphotyrosine at a final concentration of 10 nmoles/ml slows the reaction rate so that complete dephosphorylation of substrate 1 does not occur until after 7 minutes of reaction time.

### Example 3

This example describes the response of isolated mud puppy taste cells to bitter-tasting compounds and the inhibition of that response by several inhibitors of phosphatase.

Taste cells are isolated from the mud puppy, a type of salamander (S. C. Kinnamon, et al, *Chemical Senses* 13: 355 - 366, 1988). Briefly, a fresh mud puppy tongue is incubated with collagenase, the non-gustatory epithelium is peeled off the surface, and individual taste buds are gently drawn into a micropipette using a dissecting microscope at 50X magnification. The taste buds are plated into small vessels, that are coated with Cell-Tak, containing culture media. The taste cells can remain viable up to 18 days and maintain normal functions (C. Ruiz, et al, *Experimental Cell Biology of Taste and Olfaction, Current Techniques and Protocols,* A. I. Spielman and J. G. Brand, eds., CRC Press, 1995, pp. 79 - 84). The taste cells are labeled intracellularly with a marker compound that fluoresces in a dose-dependent fashion to calcium ion concentration. Since an increase in the intracellular calcium ion concentration is one of the last steps in converging biochemical signal transduction pathways for bitterness, the fluorescent marker correlates with taste cell response to bitterness (D. Restrepo, et al, *Experimental Cell* *Biology of Taste and Olfaction, Current Techniques and Protocols,* A. I. Spielman and J. G. Brand, eds., CRC Press, 1995, pp. 387 - 398). Labeled taste cells are observed microscopically using image analysis software. Using a micropipette, taste cells are exposed to bitter-tasting compounds by adding the compounds in media near the taste cell. Cells that respond to the bitter-tasting compound show an increase in fluorescence intensity. Bitter inhibitors are tested by adding them to the test solution with the bitter-tasting compound. The response of individual taste cells to bitter-tasting compounds and to bitter-tasting compounds plus inhibitor are monitored by measuring the fluorescent intensity using a microscope. Images are stored electronically and image analysis software is used to analyze the data (D. Restrepo, et al, *Biophysical Journal* 64: 1961 - 1966, 1993). These experiments show that taste cells responded to the bitter tasting compounds, denatonium benzoate, dextromethorphan, and chlorpheniramine maleate, by an increase in intracellular calcium concentration. Sodium orthovanadate, a cell permeant inhibitor of protein tyrosine phosphatase, inhibits the response of these taste cells when mixed with denatonium benzoate. However, okadaic acid, a cell permeant inhibitor of serine-threonine phosphatases, has no effect on response to denatonium benzoate or dextromethorphan. VMP decreases the taste cell response to denatonium benzoate and dextromethorphan. Phosphotyrosine decreases the response to dentatonium benzoate, dextromethorphan and chlopheniramine maleate. Pamidronic acid and phosphonic acetic acid cause a small reduction in response to denatonium benzoate and dextromethorphan in taste cells. Methylene diphosphonic acid causes a small decrease in response to denatonium benzoate in 50% of the mud puppy taste cells tested.

Sodium orthovanadate and okadaic acid are known in the scientific literature to be cell permeant and to inhibit specific types of phosphatase enzymes. The other inhibitor compounds tested were small phosphorous-containing compounds. There are several examples in the scientific literature of compounds with similar chemical structures that are capable of entering or being transported into cells. Just a few examples are: fructose-1,6-bisphosphate (B. Tavazzi, et al, *Basic Res. Cardiol.* 87: 280 - 289, 1992), amino-phosphonate vinca alkaloid analogue (C. Tolis, et al, *Anticancer Res.* 13: 161 - 166, 1993), and alendronate, a bisphosphonate (M. H. Chestnut, et al., *Uptake of Bisphosphonates by Mammalian Cell, Localization Using Light Microscopy and Fluorescently Labeled Alendronate,* 3rd Workshop on Bisphosphonates: From the Laboratory to the Patient; in *Bone* 17(6):599, (1995)). These examples demonstrate that such compounds can move to the intracellular compartment. Our experiments with isolated taste cells demonstrate that externally applied compounds alter the intracellular signal transduction biochemical cascade caused by bitter-tasting molecules.

### Example 4

This example demonstrates the human taste response to bitter-tasting compounds mixed with phosphatase inhibitors.

Many experimental designs or sensory test protocols have been used to measure the sensory taste experience. Two different experimental designs are described herein to determine whether the compounds that inhibit phosphatase enzymes and decrease the taste cell response to bitter-tasting compounds decrease the sensation of bitterness in human taste tests.

In the first taste test, the detection threshold for a bitter-tasting compound is determined by having subjects taste a series of solutions containing increasing concentrations of the bitter-tasting compound. None of the subjects are able to taste the bitterness in the first solution containing the lowest concentration of the bitter-tasting molecule. The subjects are instructed to indicate the first, or lowest concentration of solution that is sensed as bitter. This concentration is the detection threshold for that particular compound and subject. Another identical series of solutions containing the increasing concentrations of the bitter compound plus a constant concentration of VMP, the putative inhibitor, is also tested. The detection threshold for the bitter-tasting compound plus inhibitor is likewise determined. All of these aqueous solutions contain 5% sucrose. The sample without VMP contains 10 ppm vanillin to further blind the sample. The dilution series are in ¹/₂ log increments (i.e., 1 uM, 3.2 uM, 10 uM, 32 uM). The pair of dilution series with and without inhibitor is tasted within a 2 hour time period. Subjects are blinded as to the identity of the bitter compound and whether inhibitor is present or not. The two dilution series are presented in random order. If the series with VMP has a higher detection threshold than the series with only the bitter-tasting compound, then an inhibition of bitterness by VMP is demonstrated. As shown in Table 5 below, VMP increases the detection threshold for chlorpheniramine maleate, doxylamine succinate, and phenylpropanolamine.

**TABLE 5**

| Detection Threshold of Bitter-tasting Compounds and Effect of 0.3% VMP | | | | |
|---|---|---|---|---|
| **Bitter-Tasting Compounds** | **n** | **# VMP increased threshold** | **# VMP did not change threshold** | **% Subjects that VMP increased threshold** |
| chlorpheniramine maleate | 5 | 4 | 0 | 80.00 |
| doxylamine succinate | 3 | 2 | 1 | 66.67 |
| phenylpropanolamine | 7 | 4 | 2 | 57.14 |
| denatonium benzoate | 4 | 2 | 2 | 50.00 |
| cimetidine | 2 | 1 | 1 | 50.00 |
| acetaminophen | 5 | 2 | 3 | 40.00 |
| pseudoephedrine | 5 | 2 | 2 | 40.00 |
| ranitidine | 3 | 1 | 1 | 33.33 |
| guaifenesin | 8 | 2 | 2 | 25.00 |
| domiphen bromide | 4 | 1 | 3 | 25.00 |
| dextromethorphan | 5 | 1 | 4 | 20.00 |
| sucrose octaacetate | 7 | 1 | 4 | 14.29 |
| CPC | 2 | 0 | 1 | 0.00 |
| famotidine | 3 | 0 | 3 | 0.00 |
| caffeine | 4 | 0 | 3 | 0.00 |

A second taste test design uses paired comparisons with bitter-tasting compound in both samples and VMP as the inhibitor in one sample. In this test all samples contain 5% sucrose. The sample without VMP contains 10 ppm vanillin to match the vanillin flavor of the VMP and further blind the samples. Subjects rate which sample is most bitter, and also indicate on a 7 point scale the certainty of their choice. A high value for ^{correct}/ₜₒₜₐₗ and for % certainty indicates that the inhibitor is effective. The results are shown in Table 6 below. VMP is a good inhibitor for dextromethorphan and chlorpheniramine maleate. However, it inhibits bitterness of phenylpropanolamine and guaifenesin to a lesser degree.

**TABLE 6**

| Paired Comparisons of Bitter-Tasting Compounds with and without VMP Decrease of Bitterness by VMP | | | | | | |
|---|---|---|---|---|---|---|
| **Bitter Compound** | **Conc. (mM)** | **Inhibitor (0.3%)** | **Correct # responses *** | **Total # responses** | **Corr./Tot. **** | **Cert. (%) ***** |
| dextromethorphan | 0.320 | VMP | 4 | 4 | 1.00 | 82 |
| chlorpheniramine | | | | | | |
| maleate | 1.000 | VMP | 4 | 4 | 1.00 | 71 |
| chlorpheniramine | | | | | | |
| maleate | 0.100 | VMP | 4 | 4 | 1.00 | 54 |
| phenylpropanolamine | | | | | | |
| HCl | 10.00 | VMP | 4 | 5 | 0.80 | 49 |
| acetaminophen | 32.00 | VMP | 4 | 5 | 0.80 | 29 |
| guaifenesin | 10.00 | VMP | 3 | 4 | 0.75 | 57 |
| pseudoephedrine HCl | 0.320 | VMP | 3 | 5 | 0.60 | 14 |
| acetaminophen | 10.00 | VMP | 2 | 4 | 0.50 | 43 |
| guaifenesin | 3.200 | VMP | 2 | 4 | 0.50 | 36 |
| guaifenesin | 0.320 | VMP | 2 | 4 | 0.50 | 14 |
| pseudoephedrine HCl | 1.000 | VMP | 2 | 5 | 0.40 | 34 |
| sucrose octaacetate | 0.003 | VMP | 2 | 5 | 0.40 | 23 |
| phenylpropanolamine | | | | | | |
| HCl | 3.200 | VMP | 2 | 5 | 0.40 | 11 |
| sucrose octaacetate | 0.001 | VMP | 1 | 5 | 0.20 | 20 |

| | | | | | | |
|---|---|---|---|---|---|---|
| * *correct response means that the sample without VMP was chosen as most bitter* | | | | | | |
| ** *corr / tot means the ratio of correct # responses to total* # *responses* | | | | | | |
| *** *cert % is the mean certainty score of the subjects* | | | | | | |

### Example 5

This example demonstrates a typical pharmaceutical cough/cold composition comprising a bitter tasting compound and an inhibitor of intracellular phosphatase enzymes of taste cells.

A two-fold concentrated cough syrup is prepared by mixing the following three ingredients until all solids are dissolved.

| Phase A | |
|---|---|
| 1) 95% ethanol | 170.32 g |
| 2) propylene glycol | 200.0 g |
| 3) PEG 400 | 200.0 g |

The following pharmaceutical compounds are then added and the composition containing all six ingredients is mixed completely until every component is in solution.

| | |
|---|---|
| 4) acetaminophen | 66.68 g |
| 5) pseudoephedrine HCI | 4.0 g |
| 6) dextromethorphan HBr | 2.0 g |

The following four ingredients are mixed together seperate from the above composition until they are completely dissolved.

| Phase B | |
|---|---|
| 1) water | 600 ml |
| 2) sodium citrate, dihydrate | 9.32 g |
| 3) high fructose corn syrup | 1100 g |
| 4) sodium saccharin | 1.4 g |

Citric acid is added to the above four ingredients and all five ingredients are mixed until completely dissolved.

| | |
|---|---|
| 5) citric acid, anhydous | 3.516 g |

Phase B is then allowed to sit at room temperature undisturbed for approximately two hours so that all of the air is released from the solution.

Phase A and Phase B are then combined and water is added until the final volume equals 2.0 liters.

For the final cough syrup composition, 3 g of VMP is added to 500 ml of the dual phase composition described above. Water is then added to bring the final volume up to 1.0 liter and the composition is mixed throughly.

## Claims

1. A method of inhibiting an undesirable taste in oral compositions comprising inhibiting phosphatase enzymes present in taste cells by use of a composition comprising an inhibitor of said enzymes.

2. A method according to Claim 1 wherein said oral compositions are selected from foods, drinks, pharmaceuticals, toiletries, and mixtures thereof.

3. A method according to Claim 1 or Claim 2 wherein said undesirable taste is selected from bitter, metallic, and mixtures thereof.

4. A method according to any of Claims 1 to 3 wherein said taste is provided by at least one orally administrable pharmaceutical active selected from bronchodilators, anorexiants, antihistamines, nutritional supplements, laxatives, analgesics, anesthetics, antacids, H2-receptor antagonists, anticholinergics, antidiarrheals, decongestants, demulcents, antitussives, antinauseants, antimicrobials, antibacterials, antifungals, antivirals, expectorants, anti-inflammatory agents, antipyretics, and mixtures thereof.

5. A method according to any of Claims 1 to 4 wherein said inhibitor of intracellular phosphatase enzymes of taste cells is selected from phosphates, thiophosphates, phosphonates, vanadates, bisphosphates, bisphosphonates, phosphate-phosphonates, thiophosphate-phosphates, thiophosphate-phosphonates, their physiologically-relevant salts and ester derivatives, and mixtures thereof.

6. A method according to Claim 5 wherein said phosphates are selected from vanillylmonophosphate (VMP), eugenylmonophosphate (EMP), menthylmonophosphate (MMP), thymylmonophosphate (ThMP), phosphotyrosine, β-napthyl phosphate, α-napthyl phosphate, phenyl phosphate, 4-nitrophenylphosphate, 3-nitrophenylphosphate, 2-chlorophenyl phosphate, D-inositol-1-phosphate, D-3,5,6-trideoxy-inositolphosphate disodium, D-6-deoxy-inositolphosphate disodium, α-napthylyl acid phosphate monosodium, vanillyl monophosphate monosodium, ethyl vanillyl monophosphate monosodium, and mixtures thereof; said thiophosphates are selected from thio-MMP, *rac*-inositol 1-phosphorothioate, thiophosphoryltyrosyl-containing peptides, and mixtures thereof; said phosphonates are selected from phosphonoacetic acid, 2-amino-4-phosphonobutyric acid, benzylphosphonic acid, phosphonomethylphenylalanine, *rac*-inositol 1-phosphonate, D-inositol 1-methylphosphonate, 3,4-methylenedioxybenzylphosphonic acid monosodium, bis(acetoxymethyl)benzylphosphonate, and mixtures thereof; said bisphosphates are selected from *rac*-Inositol 4,5-bisphosphate, *rac*-Inositol 3,4-bisphosphate, and mixtures thereof, and said bisphosphonates are selected from pamidronic acid, hydroxymethylenebisphosphonic acid, methylene bisphosphonic acid, alendronic acid, azacycloheptane-2,2-diphosphonic acid, *p*-hydroxyphenyl-1-[1,1-ethylene-bisphosphonate] ether trisodium, and mixtures thereof.
